# EUROPÄISCHE PATENTANMELDUNG

(11) **EP 0 765 647 A2**
(43) Veröffentlichungstag der Anmeldung: **02.04.1997**
(21) Anmeldenummer: 96810645.0
(22) Anmeldetag: 30.09.1996
(51) Int. Cl.: A61F 5/56, A47G 9/00

(54) **Kopf- und Nackenkissen zur Vermeidung des Schnarchens**

(30) Priorität: 28.09.1995 CH 2738/95
(71) Anmelder: Berro AG, 4414 Füllinsdorf (CH)
(72) Erfinder: Berends, Jan J., 4414 Füllinsdorf (CH)
(74) Vertreter: Ullrich, Gerhard, Dr.

(57) **Zusammenfassung**

Ein Kopf- und Nackenkissen umfasst einen Kissenkörper (1), der aus einem mittleren Kissenteil (3) und zwei seitlichen Kissenteilen (2, 20) besteht. Auf den seitlichen Kissenteilen (2, 20) sind Polster (4, 5) angebracht, die weicher als der Kissenkörper (1) sind. Der mittlere Kissenteil (3) bildet eine längliche Erhöhung (30), die ein seitliches Abrollen des Kopfes (6) des Schläfers bewirkt, wenn dieser sich in Rückenlage begibt. Der Kopf (6) kommt schliesslich in Seitenlage auf einem der Polster (4, 5) zu liegen. Dadurch wird ein Öffnen des Mundes und somit das Schnarchen weitgehend verhindert.

## Beschreibung

Die vorliegende Erfindung bezieht sich auf ein Kopf- und Nackenkissen zur Vermeidung des Schnarchens eines Schläfers, wie es im Oberbegriff des unabhängigen Patentanspruchs 1 definiert ist.

Schnarchen entsteht dadurch, dass eine Person mit offenem Mund schläft und Luft durch den Mund zieht, so dass das Gaumensegel in Schwingung gerät. Im allgemeinen tritt Schnarchen dann auf, wenn eine Person in Rückenlage mit dem Hinterteil ihres Kopfes auf dem Kissen schläft. In dieser Schlafposition ist der Mund des Schläfers häufig geöffnet.

Das Schnarchen eines Schläfers kann meistens dadurch verhindert werden, dass er dazu gebracht wird, seinen Mund beim Schlafen geschlossen zu halten.

Aus der DE-OS-40 01 991 ist eine Vorrichtung zum Reduzieren des Schnarchens bekannt, die darauf beruht, den Kopf eines Schläfers in Rückenlage zu einem seitlichen Wegdrehen zu veranlassen, so dass er schliesslich in Seitenlage auf dem Kissen aufliegt. In dieser Kopfstellung ist der Mund des Schläfers üblicherweise geschlossen, und es tritt meistens kein Schnarchen auf. Die Vorrichtung besteht aus einem Abrollgebilde, das die Form eines Rugbyballes aufweist und etwa in der Mitte eines Kissens am Kisseninnenteil oder am Kissenbezug angebracht wird.

Diese Vorrichtung hat den Nachteil, dass sie zusätzlich zu einem Kissen produziert und dann in dieses eingesetzt und am Kisseninnenteil oder am Kissenbezug befestigt werden muss. Ausserdem erfolgt ein seitliches Abrollen des Kopfes nur, wenn dieser ungefähr in der Mitte des Kissens aufliegt.

Die bis anhin existierenden Kopf- und Nackenkissen des betroffenen Typs können somit als nicht optimal hinsichtlich der praktischen Verwendbarkeit und der Herstellungskosten bezeichnet werden. Die Erfindung hat sich daher zur Aufgabe gestellt, ein Kopf- und Nackenkissen zur Vermeidung des Schnarchens zu schaffen, das eine gute praktische Verwendbarkeit hat und kostengünstig hergestellt werden kann. Gleichzeitig soll es bequem sein und eine physiologisch richtige, gesunde Lagerung von Kopf und Nacken, ohne Abknicken des Kopfes, ermöglichen, so dass Nackenschmerzen vermieden werden.

Diese Aufgabe wird durch das erfindungsgemässe Kopf- und Nackenkissen gelöst, wie es im unabhängigen Patentanspruch 1 definiert ist. Bevorzugte Ausführungsvarianten ergeben sich aus den abhängigen Patentansprüchen 2 bis 10.

Das Wesen der Erfindung besteht darin, dass ein Kopf- und Nackenkissen einen Kissenkörper umfasst, der aus einem mittleren und zwei seitlichen Kissenteilen besteht. Der mittlere Kissenteil bildet eine längliche Erhöhung so aus, dass sie sich durchlaufend von der einen Kissenlängsseite bis zur anderen Kissenlängsseite erstreckt und ein seitliches Abrollen des Kopfes des Schläfers bewirkt, wenn sich dieser in Rückenlage begibt.

Das erfindungsgemässe Kopf- und Nackenkissen ist aufgrund seines einfachen Aufbaus kostengünstig herstellbar und praktisch gut verwendbar.

Im folgenden wird es anhand von zwei Ausführungsvarianten und unter Bezugnahme auf die beigefügten Zeichnungen detaillierter beschrieben. Dabei zeigen:

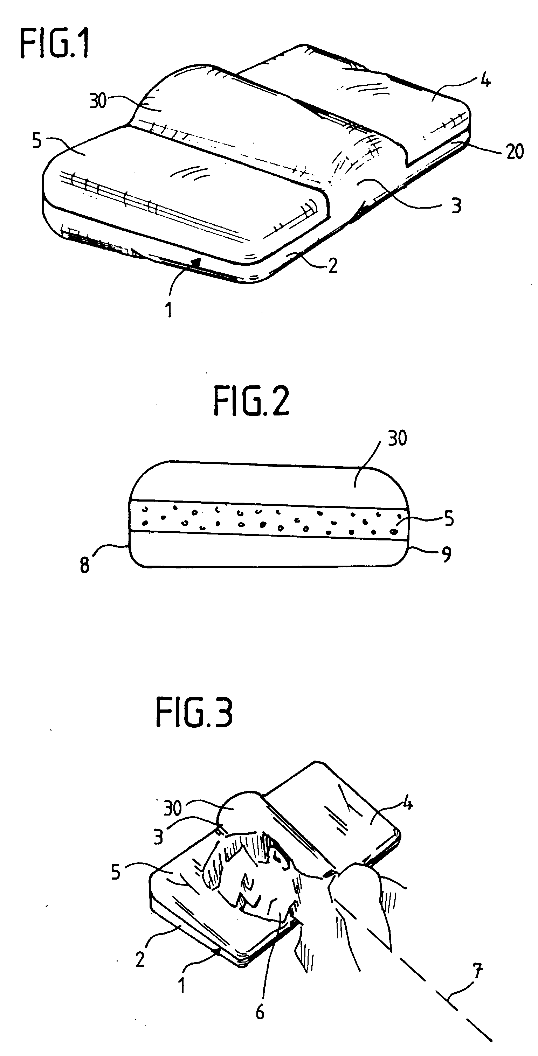

- Figur 1 -: eine Perspektivansicht einer ersten Ausführungsvariante des erfindungsgemässen Kopf- und Nackenkissens mit einem einstückigen Kissenkörper und Polstern;
- Figur 2 -: eine Seitenansicht des Kissens von Figur 1;
- Figur 3 -: die Benutzung des Kissens von Figur 1 durch einen Schläfer;

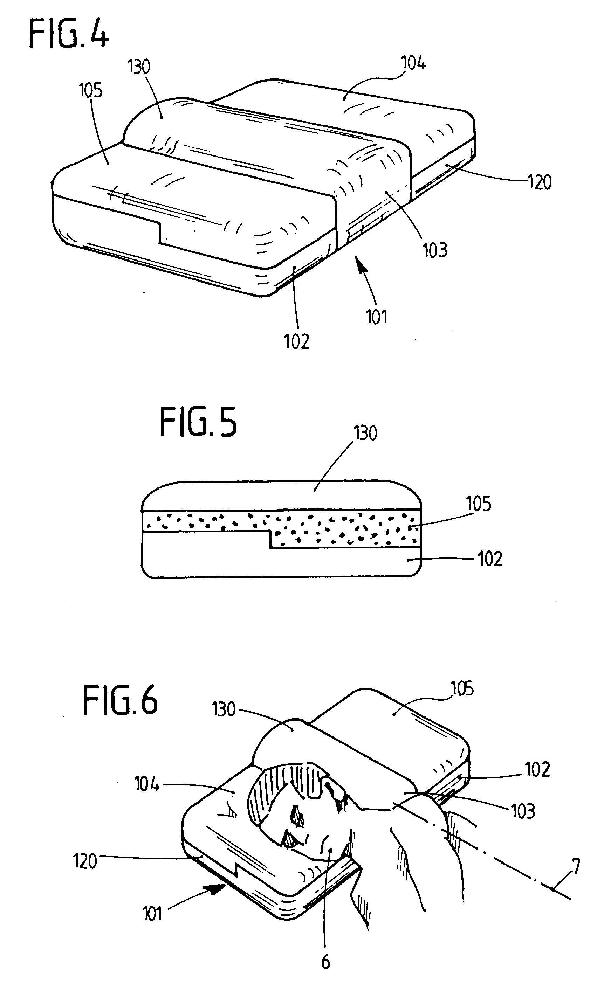

- Figur 4 -: eine Perspektivansicht einer zweiten Ausführungsvariante des erfindungsgemässen Kopf- und Nackenkissens mit stufenartig ausgebildeten seitlichen Kissenteilen und Polstern;
- Figur 5 -: eine Seitenansicht des Kissens von Figur 4 und
- Figur 6 -: die Benutzung des Kissens von Figur 4 durch einen Schläfer.

### Figuren 1 bis 3

Das Kopf- und Nackenkissen weist bei dieser Ausführungsvariante einen einstückigen Kissenkörper 1 auf, der einen mittleren Kissenteil 3 und zwei seitliche Kissenteile 2, 20 umfasst. Der untere Teil des Kissenkörpers 1, bestehend aus den zwei seitlichen Kissenteilen 2, 20 und dem unteren Teil des mittleren Kissenteils 3, ist auf der einen Kissenlängsseite 8 dicker als auf der anderen Kissenlängsseite 9, so dass das Kissen auf der Kissenlängsseite 8 höher ist als auf der Kissenlängsseite 9. Dies ermöglicht eine physiologisch richtige Lage sowohl für Leute mit breiten als auch für Leute mit schmalen Schultern. Leute mit breiten Schultern verwenden das Kopf- und Nackenkissen so, dass die höhere Kissenlängsseite 8 ihrem Körper zugewandt ist; Leute mit schmalen Schultern so, dass die niederere Kissenlängsseite 9 ihrem Körper zugewandt ist. Die seitlichen Kissenteile 2, 20 sind auf der höheren Kissenlängsseite 8 z.B. 6 cm, auf der niedereren Kissenlängsseite 9 z.B. 4 cm dick, bei einer Kissengrundfläche von z.B. 60 cm x 45 cm.

Der obere Teil des mittleren Kissenteils 3 hat die Form eines halben Zylinders, der eine längliche Erhöhung 30 bildet und sich von der einen Kissenlängsseite 8 bis zur anderen Kissenlängsseite 9 erstreckt. Der Zylinderradius ist z.B. 12 cm.

Der Kissenkörper 1 besteht beispielsweise aus Schaumstoff, vorzugsweise aus einem festeren Polyurethan.

Auf der Oberseite der seitlichen Kissenteile 2, 20 sind Polster 4 und 5 angebracht, die weicher sind als der Kissenkörper 1. Sie sind vorzugsweise mit den seitlichen Kissenteilen 2, 20 und dem mittleren Kissenteil 3 verklebt und bestehen z.B. aus 5 cm dickem Latexschaum. Nach dem durch die längliche Erhöhung 30 bewirkten Abrollen des Kopfes 6 eines Schläfers mit Körperachse 7 kommt dieser auf einem der Polster 4, 5 zu liegen. Dadurch ist das Liegen sehr angenehm, was eine gute Akzeptanz des Kopf- und Nackenkissens ergibt.

Der Kissenkörper 1 und die Polster 4, 5 werden für den praktischen Gebrauch mit einem speziellen, enganliegenden Kissenbezug (nicht dargestellt) vollständig umhüllt. Dieser Bezug ist z.B. aus Stoff, vorzugsweise Jersey, und vorteilhafterweise abnehmbar ausgestaltet.

Für die weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden Figurenbeschreibungen Bezug genommen.

### Figuren 4 bis 6

Bei dieser Ausführungsvariante besteht ein Kissenkörper 101 aus einem mittleren Kissenteil 103 und zwei mit diesem verklebten seitlichen Kissenteilen 102, 120.

Der obere Teil des mittleren Kissenteils 103 hat die Form eines halben Zylinders, der eine längliche Erhöhung 130 bildet und sich von der einen Kissenlängsseite bis zur anderen Kissenlängsseite erstreckt. Der Zylinderradius ist z.B. 12 cm. Der untere Teil des mittleren Kissenteils 103 ist quaderförmig.

Auf der Oberseite der seitlichen Kissenteile 102, 120 sind auch bei dieser Ausführungsvariante Polster 104, 105 angebracht, die weicher als der Kissenkörper 101 sind, z.B. aus Latexschaum bestehen und vorzugsweise mit den seitlichen Kissenteilen 102, 120 und dem mittleren Kissenteil 103 verklebt sind. Der Kissenkörper 101 kann wiederum aus Schaumstoff, vorzugsweise aus einem festeren Polyurethan, bestehen.

Die seitlichen Kissenteile 102, 120 sind stufenartig ausgebildet und auf der einen Kissenhälfte z.B. 6 cm, auf der anderen Kissenhälfte z.B. 3 cm dick. Die Polster 104, 105 sind ebenfalls stufenartig ausgebildet, mit Dicken von beispielsweise 6 cm und 3 cm. Sie sind so auf den seitlichen Kissenteilen 102, 120 angeordnet, dass ihr dickerer Bereich auf dem dünneren Bereich des zugehörigen seitlichen Kissenteils 102, 120 zu liegen kommt und umgekehrt. Die Kissengrundfläche beträgt z.B. 66 cm x 26 cm.

Der Kissenkörper 101 und die Polster 104, 105 werden vorzugsweise wie bei der ersten Ausführungsvariante mit einem enganliegenden Kissenbezug (nicht dargestellt) vollständig umhüllt.

Zu den vorbeschriebenen Kopf- und Nackenkissen sind weitere konstruktive Variationen realisierbar. Hier ausdrücklich erwähnt seien noch:
- Das Kissen kann eine andere Grundfläche aufweisen. Es kann z.B. rund oder oval sein.
- Die längliche Erhöhung 30 bzw. 130 kann eine andere Form aufweisen. Ihr Querschnitt kann z.B. ellipsen- oder eiförmig sein.
- Die seitlichen Kissenteile 2, 20 bzw. 102, 120 können vom mittleren Kissenteil 3 bzw. 103 aus zur zugehörigen Kissenschmalseite hin dicker werden, vorzugsweise ca. 1 cm.

## Patentansprüche

1. Kopf- und Nackenkissen zur Vermeidung des Schnarchens eines Schläfers, das auf seiner Oberseite eine ungefähr in der Richtung der Körperachse (7) des Schläfers sich erstreckende längliche Erhöhung (30; 130) aufweist, die ein seitliches Abrollen des Kopfes (6) des Schläfers bewirkt, wenn dieser sich in Rückenlage begibt, dadurch gekennzeichnet, dass es einen Kissenkörper (1; 101) umfasst, der aus einem mittleren Kissenteil (3; 103) und zwei seitlichen Kissenteilen (2, 20; 102, 120) besteht, wobei der mittlere Kissenteil (3; 103) die genannte längliche Erhöhung (30; 130) so ausbildet, dass sie sich durchlaufend von der einen Kissenlängsseite bis zur anderen Kissenlängsseite erstreckt.

2. Kopf- und Nackenkissen nach Anspruch 1, dadurch gekennzeichnet, dass der mittlere Kissenteil (3) und die beiden seitlichen Kissenteile (2, 20) einstückig ausgebildet sind.

3. Kopf- und Nackenkissen nach Anspruch 1 oder 2, dadurch gekennzeichnet, dass die längliche Erhöhung (30; 130) im wesentlichen die Form eines halben Zylinders hat und 5 bis 10 cm hoch sein kann.

4. Kopf- und Nackenkissen nach einem der Ansprüche 1 bis 3, dadurch gekennzeichnet, dass der mittlere Kissenteil (3) und/oder die beiden seitlichen Kissenteile (2, 20; 102, 120) auf der einen Kissenlängsseite dicker sind als auf der anderen.

5. Kopf- und Nackenkissen nach einem der Ansprüche 1 bis 4, dadurch gekennzeichnet, dass mindestens eines der beiden seitlichen Kissenteile vom mittleren Kissenteil aus zur Kissenschmalseite hin dicker wird.

6. Kopf- und Nackenkissen nach einem der Ansprüche 1 bis 5, dadurch gekennzeichnet, dass auf den beiden seitlichen Kissenteilen (2, 20; 102, 120) je ein im Vergleich zum Kissenkörper (1; 101) weicheres Polster (4, 5; 104, 105) angebracht ist, so dass der Kopf (6) des Schläfers nach dem Abrollen in Seitenlage auf einem der beiden Polster (4, 5; 104, 105) zu liegen kommt.

7. Kopf- und Nackenkissen nach den Ansprüchen 4 und 6, dadurch gekennzeichnet, dass die Polster (104, 105) auf der einen Kissenlängsseite dicker sind als auf der anderen, wobei sie auf der Kissenlängsseite dicker sind, auf der die beiden seitlichen Kissenteile (102, 120) weniger dick sind.

8. Kopf- und Nackenkissen nach Anspruch 7, dadurch gekennzeichnet, dass die seitlichen Kissenteile (102, 120) von der einen Kissenlängsseite aus zur anderen Kissenlängsseite hin stufenartig dicker werden, während die Polster (104, 105) in gleicher Richtung stufenartig dünner werden.

9. Kopf- und Nackenkissen nach einem der Ansprüche 1 bis 8, dadurch gekennzeichnet, dass der Kissenkörper (1; 101) aus Schaumstoff, vorzugsweise aus einem festeren Polyurethan, und/oder die Polster (4, 5; 104, 105) aus Latexschaum bestehen.

10. Kopf- und Nackenkissen nach einem der Ansprüche 1 bis 9, dadurch gekennzeichnet, dass ein enganliegender Bezug, vorzugsweise aus Jersey, den Kissenkörper (1; 101) und gegebenenfalls die Polster (4, 5; 104, 105) vollständig umhüllt.
